# EUROPEAN PATENT SPECIFICATION

(11) **EP 0 679 194 B1**
(45) Date of publication and mention of the grant of the patent: **15.09.1999**
(21) Application number: 94907825.7
(22) Date of filing: 14.01.1994
(51) Int. Cl.: C12Q 1/00, C12Q 1/02, C12Q 1/04, C12Q 1/06

(54) **METHOD TO PREDICT ACTIVE GROWTH OF BACTERIA ADHERED TO A SURFACE**
VERFAHREN ZUR VORAUSSAGE DES BAKTERIENZUWACHSES AUF EINER OBERFLÄCHE
PROCEDE DE PREVISION DE LA CROISSANCE ACTIVE DE BACTERIES COLLEES A UNE SURFACE

(30) Priority: 15.01.1993 US 4971; 11.03.1993 US 29675
(43) Date of publication of application: 02.11.1995
(60) Divisional application: 99200717.9
(73) Proprietor: REGENTS OF THE UNIVERSITY OF MINNESOTA, Minneapolis, Minnesota 55455 (US)
(72) Inventor: BLOOMQUIST, Cynthia, G., St. Louis Park, MN 55426 (US); DOUGLAS, William, Falcon Heights, MN 55113 (US); DUNNY, Gary, St. Paul, MN 55116 (US); LILJEMARK, William, Plymouth, MN 55447 (US); REILLY, Bernard, Minneapolis, MN 55414 (US)
(74) Representative: Smulders, Theodorus A.H.J., Ir.
(86) International application number: US9400567
(87) International publication number: WO9416096

(56) References cited:
- EP-A- 0 566 109
- US-A- 5 093 238
- US-A- 5 246 837
- Dialog Information Services, file 154, Medline, Dialog acc.no. 08563713, Medline acc.no. 93273713, Gelhaye E. et al: "Adhesion and growth rate of Clostridium cellulolyticum ATCC 35319 on crystalline cellulose", J Bacteriol (UNITED STATES) Jun 1993, 175 (11) p3452-8

## Description

The present invention generally relates to processes for inhibiting or enhancing growth of microorganisms. One process involves treating or controlling a bacterial infection caused by adherent bacteria attached to or on surfaces. Another aspect of this invention particularly relates to a process to treat or control infection of adherent bacteria on surfaces of teeth or internal implanted surfaces. Still another aspect of this invention relates to a method to control active or rapid growth of adherent bacteria using a composition which initiates or starts active growth of bacteria.

### Background of the Invention

Treatment and control of bacterial infection has been a principal focus of modern research. The introduction and widespread use of antibiotics has provided the medical practitioner with a variety of powerful tools to treat and control bacterial infection in patients. In spite of the vast amounts of research undertaken as well as readily available pharmaceutical agents to combat bacterial infection, the problems of treating and controlling bacterial infection are not solved. For example, the widespread use of antibiotics has led to the development of strains of pathogenic organisms which are resistant to most commonly used antibiotics. In addition, the increasing number of immune-compromised patients has created new challenges for the medical practitioner. Furthermore, medical practitioners have long recognized that the presence of indwelling foreign devices, such as dental implants, artificial heart valves or catheters, predisposes a patient to bacterial infection and complicates the treatment and control of infection related to such foreign bodies.

It is certainly expected, however, that the implantation of devices will continue to increase as advances are made in both biomaterials and implantation methodologies. In spite of this expected increase in the use of indwelling biomaterials, effective methodologies and pharmacological agents useful to treat and control bacteria associated with the use of these materials are not currently available.

The control of bacterial infestation and growth on surfaces is particularly problematic because bacterial infestation of such surfaces is not well understood. Even though bacteria are prone to infest the surface of nearly all accessible articles, little is known about control of such bacteria on these surfaces or about the factors contributing to the growth of such bacteria on such surfaces.

Mechanisms thought to be significant in development of bacterial biomass on accessible surfaces include (1) adherence of indigenous bacteria to specific receptors of enamel pellicular surfaces; and (2) growth or cell division of adherent bacteria. It is thought that growth of adherent bacteria is very slow.

It has been observed and reported that bacteria which are adhered to surfaces are significantly different from bacteria which are in a solution or which are not bound to a surface. For example, bacteria which are in solution are more susceptible to antibiotics when compared to bacteria which are adhered to surfaces. Thus, an understanding of the processes used by bacteria to grow on or adhere to surfaces is necessary to successfully treat or control bacteria infections related directly or indirectly to surfaces.

In addition, microbial growth factors important in stimulating growth in a population of cells or initiating growth in an individual cell have not been identified or well characterized. The identification of microbial growth factors from bacterial cells growing on surfaces or in suspension could be important tools for enhancing the growth of bacteria or inhibiting the growth of bacteria in suspension or on surfaces.

Clearly, there exists a need to understand and to predict bacterial growth on the surface of biomaterials as well as control the proliferation and metabolism of adhered bacteria associated with these materials. A need also exists to identify and characterize microbial growth factors so that bacterial growth on surfaces or suspension can be enhanced or inhibited.

### Summary of the Invention

The present invention provides a novel solution to the above identified problems currently associated with treating or controlling bacterial growth on surfaces because it has now been determined that bacterial growth on a surface of a material is density dependent. Unless an adherent bacterial population approaches a density associated with an enhanced proportion of actively growing bacteria to viable bacteria, bacterial growth is unsubstantial. Put another way, substantial proliferation and metabolism of bacteria which are adhered to surfaces of biomaterials are independent of both time and nutrient supply.

Thus, one aspect of the present invention provides a process to predict when bacteria will exhibit active growth on a surface which includes the steps set forth in claim 1. According to the invention, a first population density of viable bacteria on the surface is determined. Then comparing the first population density with a second population density having a stimulated or enhanced proportion of actively growing bacteria to viable bacteria in the second population, preferably at a density of about 400,000 bacteria/mm² to about 6,300,000 bacteria/mm². Active bacterial growth can be predicted when the first population density is greater than the second population. The method can preferably be used to measure the growth of bacteria on surfaces. The proportion of actively growing cells to viable cells in a population may be readily determined using the processes described in Example 1, below.

Still another aspect of this invention is a process to predict a rapid increase in the growth of bacteria on a surface which includes the steps of determining a population density of viable growing bacteria on the surface, determining a population of actively growing bacteria on the surface, computing a ratio of actively growing bacteria to viable bacteria on the surface, and predicting active bacterial growth on the surface when the ratio of actively growing bacteria to viable bacteria is between about 5 x 10⁻⁴:1 to about 20 x 10⁻²:1. Preferably, the ratio of actively growing bacteria to viable bacteria is between about 10 x 10⁻⁴:1 to 40 x 10⁻⁴:1.

The invention also includes a method for inhibiting bacterial growth on a surface by controlling bacterial growth so that the population density is maintained below the population density associated with an enhancement or stimulation of the proportion of growing cells to viable cells in the population (i.e., density dependent growth). The bacterial growth can be controlled by limiting the contact of bacteria with adherence promoting agents such as carbohydrates or by modifying the hydrophobicity of the surface.

In addition to the methods referred to above, the present invention includes methods for controlling or treating bacterial infections.

### Brief Description of the Drawings

Figure 1A is a graphic representation of active growth of bacteria on in vivo dental implants as population approaches a density of the bacteria of about 2,500,000 - 3,200,000 [10^{6.4-6.5}] bacteria/mm².

Figure 1B is a graphic representation of the saturation of enamel pellicular binding sites which occurs during the first 2 through 8 hours on in vivo dental implants. Oral flora saturate these surfaces at a density of 200,000 - 630,000 [10^{5.3} - 10^{5.8}] per mm².

Figure 2 is a graphic representation of active growth of bacteria on in vivo dental implants in the presence of sucrose, glucose or water as a bacterial population approaches a density of about 2,500,000 - 3,200,000 [10^{6.4-6.5}] bacteria/mm².

Figure 3 is a graphic representation of a comparison of total viable bacteria in the mouth on enamel and on enamel treated with sodium octadecyl sulfate.

Figure 4 is a graphic representation of active bacterial growth on the surface of bovine enamel, VISIO-DISPERS (trademark) TM light-cured composite dental restorative (ESPE- Premier, Norristown, PA) and SILUX (trademark) TM restorative (3M, St. Paul, MN) as the population density on any of such surfaces approaches about 2,500,000 - 3,200,000 [10^{6.4-6.5}] bacteria/mm².

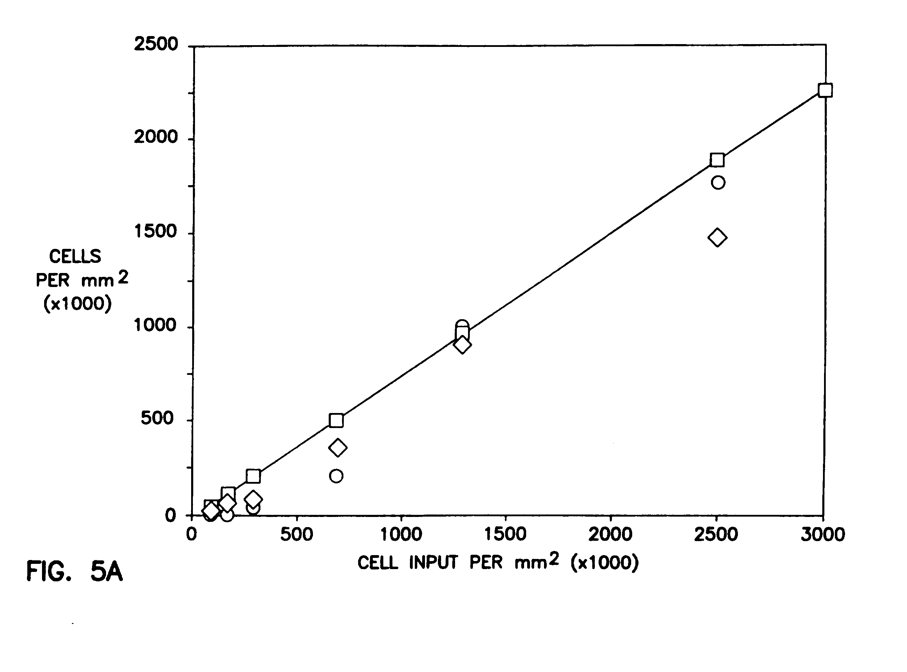

Figure 5A represents the immobilization of radio-labeled S. gordonii Challis strain ATCC Deposit No. 35105, (*) and Streptococcus parasanguis PW213(•) to maleic anhydride activated plates as a function of cell input per mm².

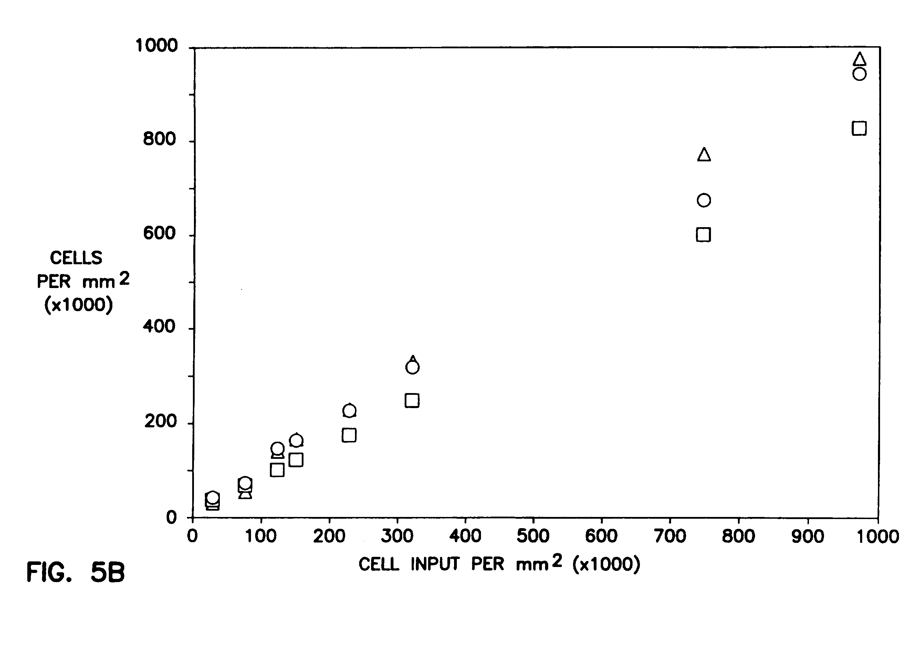

Figure 5B represents the binding of radiolabelled S gordonii Challis strain ATCC Deposit No. 35105 as a function of cell input per mm² to three surfaces: reacti-bind maleic anhydride activated polystyrene strip plates (□) (Pierce, Rockford, Ill); Immulon strip plates (◇) (Dynatech Labs) and Easy Wash ELISA strip wells (•) (Corning, NY).

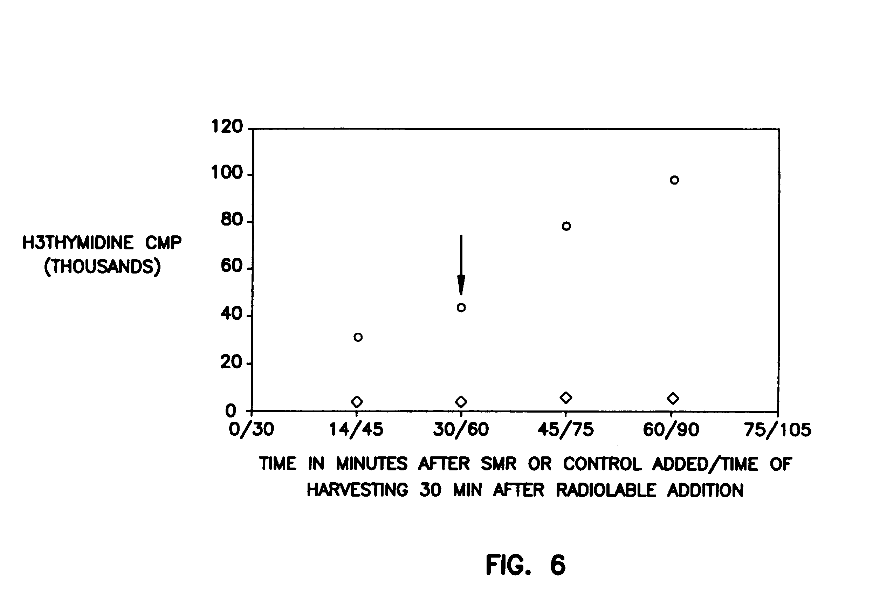

Figure 6 represents the incorporation of [³H-methyl]-thymidine into S. gordonii Challis strain ATCC Deposit No. 35105 in the presence of isolated SMR factor (*) and control Todd Hewitt (TH) media (□) as a function of the time of incubation of SMR or TH. Arrow depicts time of harvest in the preferred in vitro bacterial replication assay.

### Detailed Description

The present invention discloses processes to treat or control the growth of bacteria which are attached or adhered to surfaces of biomaterials. The present invention provides processes to determine under what conditions and at what periods of time bacteria will exhibit enhanced, stimulated, active, or rapid growth on a surface. Active growth of bacteria is directly related to population density. Density dependent growth is associated with a stimulation or enhancement of the proportion of actively growing cells in the viable cell population. The proportion of actively growing cells to the number of viable cells in a population can be determined by a number of methods, but is preferably measured by incorporation of a radiolabelled nucleotide into cellular DNA. The stimulation or enhancement of the proportion of growing cells to viable cells results in a ratio of growing to viable cells of about 5 x 10⁻⁴:1 to 20 x 10⁻²:1, preferably 10 x 10⁻⁴:1 to 40 x 10⁻⁴:1. Density dependent active growth can be achieved when a natural predominantly streptococcal population of bacteria having a size approaching 1-2 µm has a population density of approaching about 400,000 [10^{5.6}] bacteria/mm² to about 6,300,000 [10^{6.8}] bacteria/mm². Preferably, the population density approaches about 2,500,000 - 3,200,000 [10^{6.4-6.5}] bacteria/mm².

As used in this document, the following terms have the following meanings.

Viable Bacteria means living bacteria which will grow in the presence of a suitable nutrient-containing media. Typically, an aliquot or sample containing bacteria is added to a nutrient-rich agar medium and the bacteria in the aliquot or sample are grown. The number of bacteria contained in the sample may be readily determined by counting the number of colony forming units which grow on the agar. The number of viable bacteria, however, does not provide any information relating to a specific state of growth that such bacteria may be in at a given time. Thus, viable bacteria include dormant, latent, and actively growing and dying bacteria.

Actively Growing Bacteria means bacterial cells which are in a relatively brief or short period of time in which bacterial cells enter a period of active or rapid proliferation, metabolism, growth and replication compared to the rate of proliferation, metabolism, growth and replication observed for dormant, latent or slowly growing cells. Actively growing cells can be measured by looking at an increase in number of viable cells per unit of time when growing in liquid culture or suspension. When bacteria are in the exponential phase in liquid culture, a doubling time can be calculated based on the change in the numbers of viable cells per unit of time. An increase in the proportion of actively growing to viable cells in a population can be determined by measuring the proportion of cells actively synthesizing DNA or RNA or polypeptide. Total cell mass, polypeptide, RNA and DNA are functions of growth rate. See Physiology of the Bacterial Cell: A Molecular Approach, eds. F.C. Neidhardt et al. (1990) Sinauer Associates, Inc. at pages 199-202, 216-220. The proportion of actively growing cells can be measured by measuring the amount of cellular DNA synthesis with the labelling technique as described in Example 1.

Pathogenic Bacteria means any genera, strain or type of bacteria which will have a detrimental clinical impact on a patient where the detrimental effect is due to undesired bacterial growth or to the presence of undesired bacterial metabolites. Pathogenic bacteria are known to those of skill in the art and have been described in reference textbooks such as Medical Microbiology: An Introduction to Infectious Diseases, 2d Edition, John C. Sherris (editor) (1990) Elsivier Publishing Co., Inc., New York, NY. It is understood that the pathogenicity of a specific genera, strain or type of bacteria will vary from patient to patient. One of ordinary skill in the art, however, may readily determine what specific bacteria detrimentally impact a specific patient without having to undergo undue testing or experimentation. It is also understood that not all bacteria will detrimentally affect a patient and that the presence of known, relatively benign or innocuous bacteria (i.e., normal flora) on biomaterial surfaces is not considered to be detrimental and may be beneficial to a patient.

Opportunistic Bacteria are those microorganisms that are usually considered non-virulent or of limited virulence but that can cause disease or infection if they reach protected areas of the body or if the general host defense mechanisms are compromised. Opportunistic bacterial infections are known to those of skill in the art and have been described in textbooks such as Medical Microbiology, cited supra.

Population Density means the number of viable bacteria which are adhered or attached to a specific area of a surface or in a known volume of a liquid. The population density of such bacteria may be determined using well known culturing, plate counting methods and vital stains.

Biomaterial means articles having surfaces where viable bacteria may adhere, attach or stick. Suitable biomaterials include a variety of materials such as resins, restoratives, composites, metals or ceramics. Surfaces includes the exposed surfaces of materials which may contact bacteria contained in fluids.

Adherence Promoting Agents means materials, substances or compositions which promote or enhance the ability of bacteria to adhere, attach or stick to a surface.

Adherence Suppressing Agents means materials, substances or compositions which may be used to modify the surface properties of a material in order to suppress or inhibit the ability of bacteria to adhere, attach or stick to a biomaterial surface.

Dynamic Contact Angle means a measurement of the interaction of a surface or modified surface with water using a Wilhelmy balance according to the methodology set out by the manufacturer (the Wilhelmy balance is commercially available form Cahn Instruments, Model DCA-332, Cerritos, CA). The relative hydrophobicity of a surface or modified surface may be determined by this method.

Immobilized Bacteria means bacteria which are linker attached to a surface. Immobilization can include binding of bacteria to a surface using chemical or biological agents such as maleic anhydride or support bound antibodies. Adherent bacteria means bacteria attached to compatible surfaces using endogenous adhesins. Bound bacteria means bacteria associated with surfaces.

### Methods of Predicting Active Growth of Bacteria on a Surface

The present invention discloses that the proliferation and metabolism of bacteria which are bound to surfaces are independent of both time and nutrient supply and that such adhered bacteria will not significantly proliferate or metabolize unless the bacterial population approaches a population density associated with density dependent growth on natural surfaces.

In view of the fact that bacteria are found in essentially every part of the natural world and that when bacteria are present they inevitably attempt to adhere to accessible surfaces, methods and processes for controlling such bacteria have a wide range of applicability. Specifically, surfaces which are accessible to bacteria may include dental biomaterials, indwelling medical devices, other dental and medical instruments which come in contact with fluids such as sera, body fluids, other polypeptide-, glycoprotein- or carbohydrate-containing fluids as well as free-living bacteria. Surfaces found in the home, work and industrial environments are susceptible to adherence of bacteria.

The examination of biomaterials found in the mouth or oral cavity serves as a readily available model to study the behavior and properties of bacteria adhered to surfaces. Surfaces contained in the oral cavity are readily accessible and the oral cavity includes a wide range of bacterial genera and species which have been found to be detrimental in other protected areas of the body. For example, it has been reported that certain bacteria which are present in the oral cavity are capable of opportunistically infecting the endocardial surface of the heart which normally is a protected area and is usually sterile, i.e. is not typically contaminated by or with bacteria. This type of endocardial infection is especially problematic for patient's implanted with prosthetic heart valves. In view of the presence of the multiple bacteria genera and strains throughout the mucosal surfaces of the body as well as generally related environments, it is expected that methods and processes used to evaluate, treat and control bacteria adhered to surfaces in the oral cavity will readily transfer to the evaluation, treatment and control of bacteria adhered to surfaces in other parts of the body or the environment.

Furthermore, it is also expected that surfaces accessible to bacteria which contact sera or other body fluids also exhibit density dependent growth phenomena similar to the behavior of bacteria found in the oral cavity. Additionally, the methods and processes disclosed in this specification will be adaptable to evaluate, treat and control bacteria adhered to surfaces outside of either the oral cavity or body.

An important factor to consider in the development of methods and processes which impact on bacteria is the need to selectively target pathogenic or opportunistic bacteria without detrimentally affecting benign or beneficial bacteria associated with normal flora. The efficacy of antimicrobial agents may be significantly enhanced by contacting pathogenic bacteria with both a specific antimicrobial agent and start microbial replication (SMR) factor because it is well known that bacteria are particularly susceptible to antimicrobial agents when they are in a period of active growth but may be unaffected by antimicrobial agents when they are in a dormant or latent growth period.

One method for targeting growth of pathogenic or opportunistic bacteria is to contact bound bacteria with a specific antimicrobial agent in the presence of a start microbial replication factor. Without being meant to limit the invention, it is thought that adherent bacteria are nonresistant to antimicrobial agents, in part due to slow growth rates. Gustina et al., Biomaterials, 8:423 (1987) and Brown et al., Antimicrobial. Chemotherapy, 22:777 (1988). Growth rate is known to be the primary modulator of antibiotic action. Pathogenic bacteria can preferably be targeted by contacting the bacteria with an antimicrobial agent specific for the type of pathogenic or opportunistic bacteria and a microbial replication factor as described herein. While not meant to limit the invention in any way, it is believed that the stimulation of active growth would increase the proportion of pathogenic or opportunistic bacteria that are susceptible to the antimicrobial agent. Antimicrobial agents specific for pathogens and opportunistic bacteria are known to those of skill in the art and are described in Internal Medicine, 4th Ed., Editor in Chief Stein, Mosby-Year Book Inc., St. Louis, Missouri or Wilson et al., Harrison's Principles of Internal Medicine, 12th Ed., Vol. 1, Eds., McGraw-Hill, Inc., New York, NY.

Alternatively, it may not be necessary to take into account the presence of benign bacteria or normal flora in selecting an antimicrobial agent as certain protected areas of the body are considered sterile, i.e. not typically contaminated by or with bacteria. These protected areas of the body include the interior of the eye, the pericardial sac including the heart, internal organs, bones and muscles, and the brain. For controlling pathogenic or opportunistic infection in these protected areas of the body, an antimicrobial agent including a broad spectrum agent can be administered with a microbial replication factor.

Methods of the invention also include predicting when bacteria bound to a surface will exhibit active growth. The method involves determining a density at which the proportion of growing cells to viable cells in the population is increased to about 5 x 10⁻⁴:1 to about 20 x 10⁻²:1. An enhancement or stimulation of actively growing cells in a population of bacteria is density dependent. Once this density is determined, one can predict when a population of bound bacteria will exhibit active growth. The proportion of actively growing cells to viable cells in populations treated with various agents such as microbial replication factors can be compared.

The determination of population density of viable bacteria bound to surfaces may be accomplished using a variety of methods and processes. For example, the bacteria may be removed from a surface, appropriately diluted, placed on suitable media, incubated and counted using well known methodology. Alternative measurements of determining population densities are known to those of skill in the art. This allows the determination of viable bacteria population densities which is the most accurate dental plaque population density measurement to date.

A method for determining the proportion of actively growing cells in a population contacts an aliquot of a sample concurrently measured for viable counts per measured area, with an excess of labeled nucleoside. Typically, the length of contact time is less than the minimum cell division time. Incubating bacteria in the presence of the labeled nucleoside will result in incorporation of the label into the DNA (and/or RNA) of those viable cells which are actively growing. The amount of incorporated labeled nucleotide may then be used to determine the number or portion of actively growing bacteria compared to the viable bacteria in the sample. The ratio of active growth based on viable count can be compared between samples of different viable cell surface densities.

The labeled nucleotide may be selected from suitable purine and pyrimidine derivatives which have been modified to contain an appropriate label. Suitable labels may include radioactive ligand as well as dyes or other labels known to the skilled practitioner. A preferred labeled nucleotide is a mixture of [methyl-³H]-thymidine and [C¹⁴]-adenine.

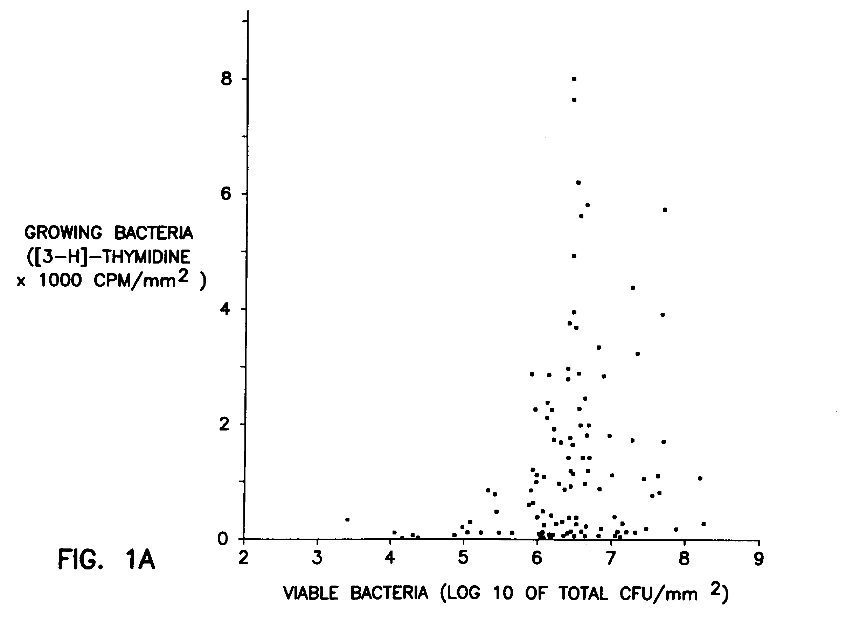

The incorporation of a labeled nucleotide, [methyl-³H]-thymidine, into viable bacteria is illustrated in Fig.1A. In this figure, the number of actively growing bacteria increased greatly as the viable cell density of 2,500,000 - 3,200,000 [10^{6.4-6.5}] was approached. At densities less than 630,000 [10^{5.8}] or greater than 6,300,000 [10^{6.8}] there was a low level of actively growing bacteria. Example 1 contains a description of the processes and techniques used to generate the data shown in Figure 1A.

In another method of the invention, bacterial growth on the surface can be controlled by maintaining a population of bacteria below a density associated with stimulation or enhancement of the proportion of actively growing cells to viable cells. Various surface treatments can either enhance or inhibit adherence and/or growth of bacteria on the surface. Inhibition of adherence and/or growth of bacteria to surfaces can be obtained by limiting the contact with adherence promoting agents such as polypeptides, glycoproteins and carbohydrates. Alternatively, growth and/or adherence of bacteria to surfaces can be inhibited by coating the surface with a hydrophobic substance, preferably a substance that creates a surface having a mean dynamic contact advancing angle in the range of about 60-130° and a mean dynamic contact receding angle greater than about 20°. Suitable examples of such compounds include commercially available products such as SILUX (trademark) restorative (3M, St. Paul, MN) and VISIO-DISPERS (trademark) composite (ESPE-Premier, Norristown, PA) and agents disclosed in U.S. Patent No. 5,078,988 issued January 7, 1992.

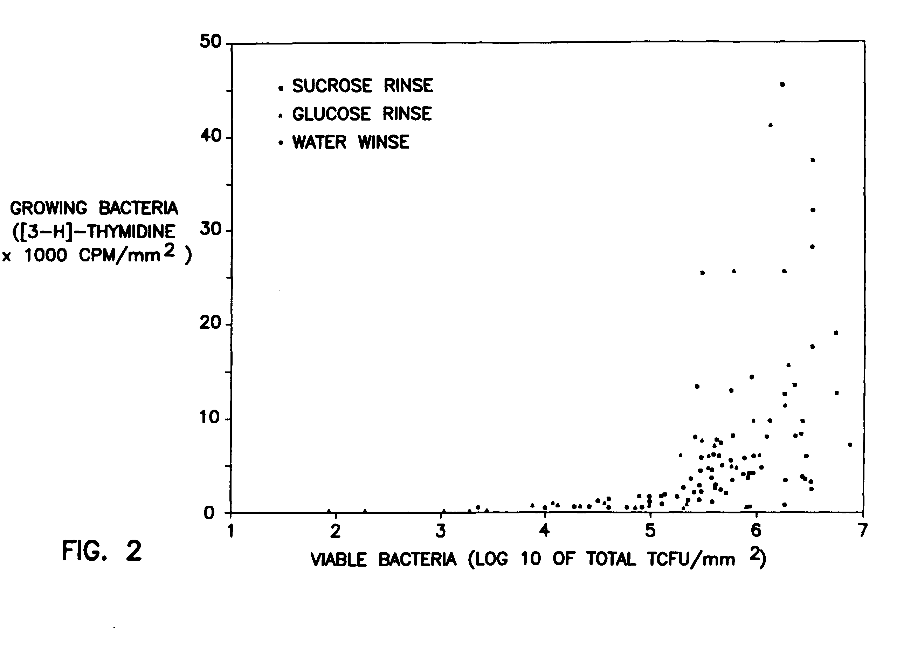

For example, Figure 2 graphically illustrates the incorporation of [methyl-³H]-thymidine into actively growing bacteria taken from implanted bovine enamel surfaces. These implants were exposed to water, glucose or sucrose rinsing in vivo. Sucrose is known to be an adherence promoting agent. As the population density of 2,500,000 - 3,200,000 [10^{6.4-6.5}] is approached, the number of actively growing bacteria dramatically increased. As is described in Example 2, this data indicate that the increase in the rapid growth of bacteria is dependent on density and independent of external factors such as sucrose, glucose or water. Adherence promoting agents can accelerate the time that a population density is reached but do not significantly affect the density at which an increase in rapid growth is seen.

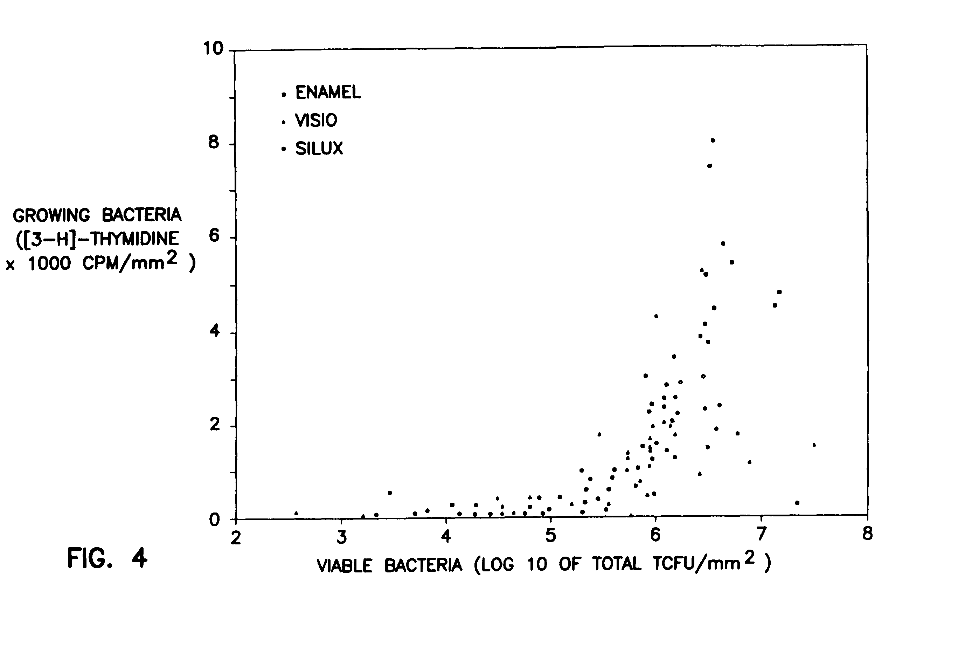

In another example, Figure 4 graphically illustrates the incorporation of [methyl-³H]-thymidine into actively growing bacteria taken from enamel, VISIO-DISPERS (trademark) or SILUX (trademark) restorative materials concurrently placed in the mouth. VISIO-DISPERS (trademark) or SILUX (trademark) are of materials having hydrophobic surfaces. Again, as the population density of 2,500,000 - 3,200,000 [10^{6.4-6.5}] is approached, the number of actively growing bacteria dramatically increased. As described in Example 4, these data indicate that the increase in rapid growth of bacteria is independent of surface, and dependent on viable cell density. However, hydrophobic surfaces can inhibit adherence and/or growth of bacteria by inhibiting the number of bacteria adhering to the surface and, therefore, the population density may remain below the level associated with density dependent active growth.

In the various embodiments of this invention, the population density of pathogenic or opportunistic bacteria on a surface is treated or controlled to maintain the population density of the bacteria below the density where density growth is observed. Density dependent growth for a bacteria having a size of about 1-2 µm is a density approaching the range of about 400,000 [10^{5.6}] bacteria/mm² to about 6,300,000 [10^{6.8}] bacteria/mm² and, preferably, a density of about 2,500,000 - 3,200,000 [10^{6.4-6.5}] bacteria/mm². While not intending to be limited by theory, it is presently believed that as a bacterial population approaches a population density associated with density dependent growth, there is a communication between the individual bacteria which initiates the onset of active growth in the population. When the bacterial population density is maintained below the density associated with density dependent growth, the prerequisite communication is not sufficient to effect concerted growth of bacteria. Thus, until a density associated with density dependent growth is approached, the detrimental effects of pathogenic or opportunistic bacteria due to growth or metabolic agents are insignificant or maintained at or limited to acceptable levels.

### Examples

The following examples are provided to further illustrate specific embodiments of this invention. These examples are intended to further describe the present invention but should not be used to limit the scope of the appended claims.

### EXAMPLE 1 - Determination of Actively Growing Populations On Enamel Inserts

Bacterial colonization of teeth in the mouth of 20 adults was studied using an in vivo model. Plaque was examined in healthy adult subjects who continued with their normal diet. (Selection criteria included the absence of systemic disease, pregnancy or extensive restorations. The subjects were instructed to maintain a normal diet and oral hygiene; excluding brushing the actual bonded test materials (see below) or rinsing with any commercial mouth rinse.)

Bovine enamel pieces having a measured area of about ten mm² were directly fixed to six contralateral upper premolars and first molars (tooth numbers 1·4, 1·5, 1·6, 2·4, 2·5, and 2·6) within custom enclosures, so that the enamel pieces could be easily inserted and removed using established methods. Enamel piece construction and enclosure in vivo are described in detail below. Following placement on 6 teeth per subject, the pieces of enamel were left for varying time periods to accumulate dental plaque. The enamel pieces covered with bacterial plaque were removed from the mouth after 2, 4, 8, or 24 hours and examined for viable microbial composition, total viable bacteria, and the proportion of the population actively growing using methods described in detail below.

These data generated information on the number of viable cells, the composition of microbial species of those viable cells, a measure of the portion of the population actively growing. Colonization of implanted surfaces, or biofilm formation, is defined as specific adherence plus cell division. Mechanisms suggested to be significant to the eventual plaque bacterial biomass include (1) adherence of indigenous bacteria to specific receptors on enamel pellicular surfaces; (2) coaggregation or adherence between bacteria already adherent to enamel (preformed plaque) and other indigenous bacteria (interbacterial adherence or coaggregation); and (3) growth or cell division of naturally adherent bacteria. Only the first mechanism has been directly studied in vivo.

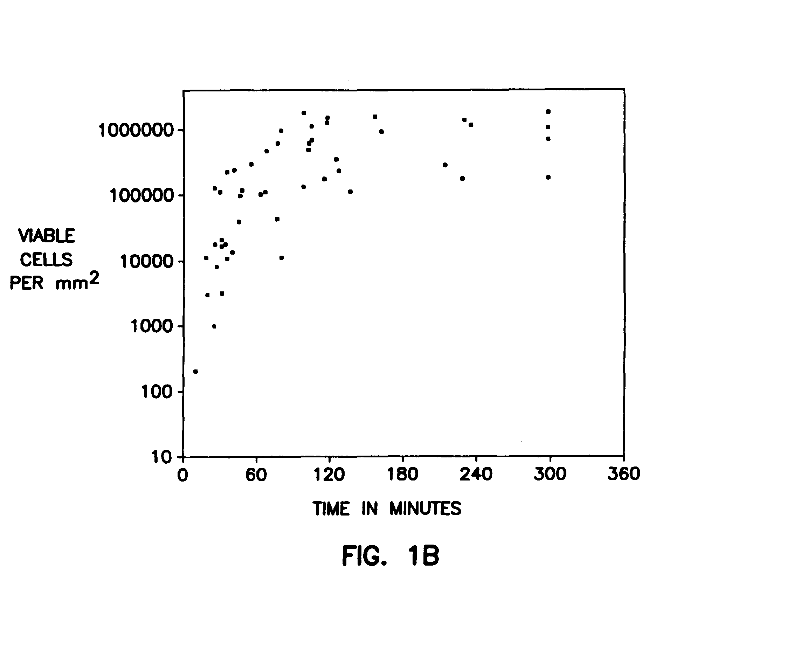

Based on data from these studies, natural adherence can be evaluated in two ways. First, the mean viable counts per mm² has been reported. Liljemark et al., Oral Micro. & Immun., 8:5-15 (1993). After 4 hours in the mouth, about 1-2,000,000 viable cells/mm² are found. The second method of evaluating the contribution of adherence to plaque biomass is exhibited in Figure 1B. By examining each tooth location in 18 subjects, saturation of pellicle binding sites can be observed. This graphical representation shows that the pellicle sites are saturated between 200,000 and 630,000 [10^{5.3} to 10^{5.8}] viable cells/mm². This corresponds to in vitro measurements of available salivary binding sites for the oral streptococci.

Based on data from similar studies, the in vivo interbacterial adherence rate was measured by inserting enamel implants covered with a continuous surface of streptococci (a strain reported to display most of the recognized coaggregation surface adhesins which cause in vitro inter-species bacterial clumping in buffer). Skopek et al., Oral Micro. and Immun., 8:16-23 (1993). Similarly, the in vivo adherence rate to implants was measured. Liljemark et al., cited supra.

Given that about 33 million bacteria per mm² form the plaque biomass by 24 hours, it is apparent that cell division is a major contributor to plaque biomass in the first day of plaque formation. Adherence at saturation to specific sites on pellicle accounts for only 1.5% of the 24-hour plaque biomass. Adherence to enamel or due to interbacterial adherence accounts for about 10-20% of the plaque biomass by 24 hours in the mouth.

These measurements of the accumulation of indigenous bacteria do not measure whether plaque bacteria were actually dividing. Growth of bacteria can be studied and is apparent when the relationship between the actively growing proportion of the plaque population is graphically illustrated based on viable cell density of individual sites (see Figure 1A). At a density approaching 2,500,000 - 3,200,000 [10^{6.4-6.5}] bacteria/mm², independent of the length of time it took to reach this density, there was a significant increase in the number of actively growing cells. This large increase in the number of actively growing bacterial cells at this population density indicates that adherent bacteria do not divide or grow slowly, but instead enter a period of rapid growth.

The method to construct and enclose enamel implants in vivo were as follows. Enamel pieces from extracted bovine teeth were cut into squares about 3 x 3 x 1 mm with 45° divergent walls. The pieces were measured using a SHERE-TUMICA digital caliper (Shere-Tumica, St. James, MN) and stored in distilled water at 4°C until used. Custom made enclosures were fabricated on a stone model of an alginate impression of the maxillary arch of each subject. The enamel pieces were pressed into a ball shaped piece of VISIO-FIL composite (ESPE-Premier, Norristown, PA) which was applied to the facial surface of the maxillary premolar and molar teeth. The composite was formed to be smooth and continuous with the enamel of the tooth and the enamel piece seated into the composite so that only its surface was exposed. The enamel pieces were then placed into the enclosures which were removable utilizing the tip of a sharp explorer via a minute notch made during fabrication of the enclosures. The enamel pieces fitted tightly into their respective enclosures preventing plaque formation anywhere except on the exposed surface. The custom enclosures were then applied to the respective teeth using a CONCISE orthodontics bonding system (3M, St. Paul, MN).

The methods to measure microbial composition and total viable cells were as follows. The enamel pieces were placed in 1.0 ml prereduced anaerobically sterilized (PRAS) diluent (L. V. Holdman and W. E. C. Moore, Eds. Anaerobic Laboratory Manual, Virginia Polytechnic Institute and State University, Blacksburg, VA, 124-127, 1973)) in an anaerobic chamber (Coy Anaerobic Chamber, Ann Arbor, MI) and gently sonified for 20 seconds (Kontes Cell Disruptor; Kontes, Vineland, NJ). An aliquot of the plaque sample was serially diluted and plated (Spiral Systems, Inc., Cincinnati, OH).

Cultivation of the various bacterial species was accomplished on a variety of elective and selective media. Total cultivable anaerobic microbial flora was determined using supplemented blood agar (SBA) medium containing Trypticase soy agar (BBL Microbiology Systems, Cockeysville, MD), 5% defibrinated sheep blood, 0.00005% menadione, 0.0005% hemin and 0.001% NAD (Sigma Chemical Company, St. Louis, MO) and incubated anaerobically at 37°C for seven days. Dark-pigmented anaerobic rods, including the species Porphyromonas gingivalis, Prevotella intermedia and Prevotella melaninogenicus, were enumerated on SBA media. Fusobacterium spp. were identified as round, entire, smooth iridescent colonies on SBA media. Streptococcus spp. were enumerated on Mitis salivarius (MS) agar (Difco Labs, Detroit, MI) with 0.1% potassium tellurite (Difco Labs, Detroit, MI) added. These plates were incubated anaerobically in an atmosphere of 10% hydrogen - 10% carbon dioxide - 80% nitrogen at 37°C for 2 days and then for 24 hours aerobically at room temperature. Adherent hard colonies, which would include the species Streptococcus sanguis, Streptococcus gordonii, and Streptococcus oralis, and referred to as sanguis streptococci, were identified visually by colonial morphology using a stereomicroscope and indirect light according to known methods. Mitis streptococci were round, smooth and creamy and included the species Streptococcus mitis and non-adherent S. oralis colonies.

Mutans streptococci were enumerated on MS agar and on MS-bacitracin media using well known methods. The colony counts of mutans streptococci shown were the ones highest on either of the two media used. Veillonella spp. were enumerated on the modified medium of Rogosa-SL agar (Difco Labs, Detroit, MI) and identified by colonial morphology and gram stained smears. Haemophilus spp. were enumerated on modified chocolate (MC) agar, following incubation in 10% carbon dioxide at 37°C for 3 days according to known methods (Liljemark et al, Infect Immun. 46: 778, 1984). Actinomyces spp. were enumerated on CFAT agar (Zylber and Jordan, J. Clin. Microbiology, 15:253-259, 1982). Representative colony types from each subject were first tested for reaction to catalase. Subsequently these strains were reacted with antisera kindly provided by Dr. George Bowden (University of Winnipeg, Winnipeg, Canada). Utilizing this system Actinomyces were speciated into Actinomyces viscous (resembling Actinomyces naeslundii II), Actinomyces naeslundii I and Actinomyces odontolyticus. Total cultivable anaerobic microbial flora (viable cells) and total colony-forming units of species were calculated per mm² surface.

The actual number of actively growing bacteria on the pellicular surface was measured using the incorporation of radiolabelled [methyl-³H]-thymidine and [C¹⁴]-adenine. To this end, plaque samples were incubated for 30 minutes, and the radiolabelled target molecules DNA and/or RNA were extracted from the plaque samples, and counted. As DNA synthesis occurs throughout the life cycle of a bacterial population, these nucleotides will label all actively growing cells. Labelling DNA is also useful as it does not turn over and because synthesis does not occur in non-growing cells. By calculating the amount of incorporation of [methyl-³H]-thymidine and dividing by the total number of viable cells, cpm/bacteria can be calculated and this ratio will reflect a change in the number of actively growing cells as a proportion of the total viable cells. Incorporation of radiolabelled nucleotide into TCA insoluble materials ensures measurement of actual DNA synthesis rather than metabolism of radiolabelled nucleotides. The short period (compared to minimum cell division time), use of excess [methyl-³H]-thymidine and [C¹⁴]-adenine and the repeated sampling over time in a manner not disruptive to plaque formation was found to be a novel method to determine growing bacterial populations.

The measurement of actively growing population are as follows. The incorporation of [methyl-³H]-thymidine and [C¹⁴]-adenine into trichloroacetic acid (TCA) insoluble material of the timed bacteria plaques was done by mixing an aliquot of sonified plaque (0.3 ml) with 0.1 ml of a mixture of [methyl-³H]-thymidine (45,000 mCi/mmol, Research Products International Corp., Mt. Prospect, IL) and [C¹⁴]-adenine (270 mCi/mmol, Amersham Research Products, Arlington Heights, IL). The final concentration of nucleosides when mixed with the plaque samples was 150 mCi/ml (100-200 nmoles) [methyl-³H]-thymidine and 65 mCi/ml (200-250 nmoles [C¹⁴]-adenine). The plaque and radiolabelled nucleosides were mixed and incubated within the anaerobic chamber for 30 minutes. Radiolabelled plaque samples were removed from the chamber, incubation terminated by the addition of 2.0 ml of cold (4°C) 10% (wt/vol) TCA and extracted for an additional 30 minutes at 4°C. The precipitate was collected on Gelman GN-6 cellulosic filters (Gelman Sciences, Ann Arbor, MI) and rinsed 3 times with 5.0 ml of cold 10% TCA. The filters were placed in vials, dried and scintillation cocktail added for counting. Radiolabel incorporation was expressed as [³H] or [C¹⁴] counts per minute (CPM) per mm² of enamel surface or per bacteria (as determined by total CFU). Radiolabel was in excess. For example, if an Escherichiacoli cell contains 9 x 10⁻¹⁵ grams of DNA per cell and 25% of the DNA is thymidic residues, then over one million times the genomic equivalent of thymidine of 10^{6.0} bacteria was added to each reaction mixture. A million excess genomic equivalents of adenine were added. The pulse labeling avoided the difficulties of determining intracellular specific activity and isotopic equilibration because each sample was pulsed for the same short time period.

Incorporation of [methyl-H³]-thymidine into bacterial cells at the density where saturation of pellicular binding sites occurred (about 200,000 - 630,000 [10^{5.3} - 10^{5.8}]) was about 3.7 x 10⁻⁴ cpm/bacteria. The total cpm of [methyl-H³]-thymidine per viable cell at this time of active growth was on the average of about 20 x 10⁻⁴ cpm/bacteria. When bacterial cells in the plaque samples are given a steady source of energy for growth, such as sucrose rinse, the cpm/bacteria was 160 x 10⁻⁴ at a density approaching about 2,500,000 to 3,200,000 cells [10^{6.4-6.5]}. See Example 2.

**TABLE 1**

| **Comparison of** ^{**3**}**H-thymidine Incorporation as a Measure of DNA Synthesis in Streptococci In Vivo and In Vitro** | | |
|---|---|---|
| | **Density per mm**^{**2**} | **(**^{**3**}**H-thymidine cpm/ bacteria) x 10**^{**-4**} |
| Overnight culture in Todd Hewitt media | [10⁹ bacteria per ml. liquid] | 2.5 |
| Predominantly streptococci adhering to enamel at saturation^{a} | 200,000 - 630,000 | 3.7 |
| | | |
| Bacteria in dental plaque^{b} on enamel, SILUX (trademark) or Visio-Dispers (trademark) | 2,500,000 -3,900,000 | 20 (160 with sucrose rinse) |
| | | |
| Bacteria immobilized to maleic anhydride plates and media^{c} | 625,000 | 5.4 |
| | | |
| Bacteria immobilized to maleic anhydride plates and START^{c} | 625,0000 | 80 |

| | | |
|---|---|---|
| ^{a} in vivo data, mean 80% of 16 sites | | |
| ^{b} in vivo data, mean 80% of 22 sites | | |
| ^{c} in vitro assay, mean 80% of 35 experiments | | |

### EXAMPLE 2 - Examining the Effects of Rinsing with Sucrose Glucose or Water on Cell Growth

Eight healthy adults, 4 males and 4 females, participated in the study. Bovine enamel pieces were mounted on the maxillary first molars and first and second premolars according to the methods described in Example 1.

Experiments were conducted for 6 hours on all 8 subjects with 6 enamel pieces per subject. Each subject rinsed her mouth with 10 ml of either water, 10% glucose (sucrose free glucose w/v) or 10% sucrose (w/v) (Sigma, St. Louis, Missouri ) every 15 minutes, for 1 minute, for the 6-hour duration of the experiment. On day one subjects rinsed with glucose, on day two with water, and on day three with sucrose. After 2 hours in vivo, 2 of the enamel pieces were randomly removed from their enclosures. After 4 hours, 2 more enamel pieces were removed as above and, after 6 hours, the final 2 pieces were removed and processed. Immediately after removal from the mouth the plaque samples were processed for viable cell counts composition described in Example 1.

Sucrose is a known adherence promoting and bacterial growth promoting substance. It was expected that in the presence of excess sucrose, that the indigenous bacterial population would immediately show a high level of active growth as they adhered to enamel implants. It was unexpected to find that, similar to indigenous plaques described in Example 1 under conditions of normal diet, that the viable adherent bacteria increased their level of active growth as they approached the density of 2,500,000 - 3,200,000 [10^{6.4-6.5}]. It is not unexpected that the level of active growth exceeded that of enamel implants without rinsing. What is unexpected is that this increase occurred as the viable cell density approached 2,500,000 - 3,200,000 [10^{6.4-6.5}]. Additionally, it was unexpected that both glucose and water rinsing increased the number of actively growing cells.

### EXAMPLE 3 - Control of Population Density Using an Adherence Suppressing Agent

Bovine enamel chips were mounted on teeth as previously described in Example 1. Solutions of octadecyl sulfate, sodium salt (Aldrich Chemicals Co., Milwaukee, WI) were formed at 60°C from deionized water and weighed proportions of sodium octadecyl sulfate (SOS) sufficient to make 5 X 10⁻³ M. The actual concentration may be somewhat less than 5 X 10⁻³ due to the solubility of SOS in water at room temperature. Only the clear supernatant was used.

Enamel pieces were incubated with the clear supernatant of SOS for 30 minutes and then washed for 5 minutes with water to remove excess solution, leaving only the oriented monomolecular layer of octadecyl sulfate. Enamel pieces with bound SOS are called SOS-enamel.

Whole saliva, stimulated with parafilm, was collected from two persons and clarified by centrifugation at 10,000 x g for 10 minutes. One mL of the clarified and mixed saliva was then incubated with enamel pieces or with SOS-enamel pieces and incubated at room temperature for 45 minutes with gentle shaking. Controls were incubated with phosphate buffer. Gibbons and Etherden, Infect. Immun., 36:52-58 (1982). The saliva-coated enamel pieces (SC-enamel) and saliva-coated SOS-enamel pieces (SC-SOS-enamel) were washed also with phosphate buffer. Gibbons and Etherden, cited supra.

Enamel, SOS-enamel, SC-enamel and SC-SOS-enamel were assessed for hydrophobicity in a Wilhelmy Balance (Cahn Instruments, DCA-322, Cerritos, CA). The piece perimeter was 17.84 mm. Time interval of immersion was one second. Platform speed was 148.39 microns/sec. The dynamic contact angles for enamel and enamel treated with SOS, and SC-SOS-enamel are illustrated in Table 2.

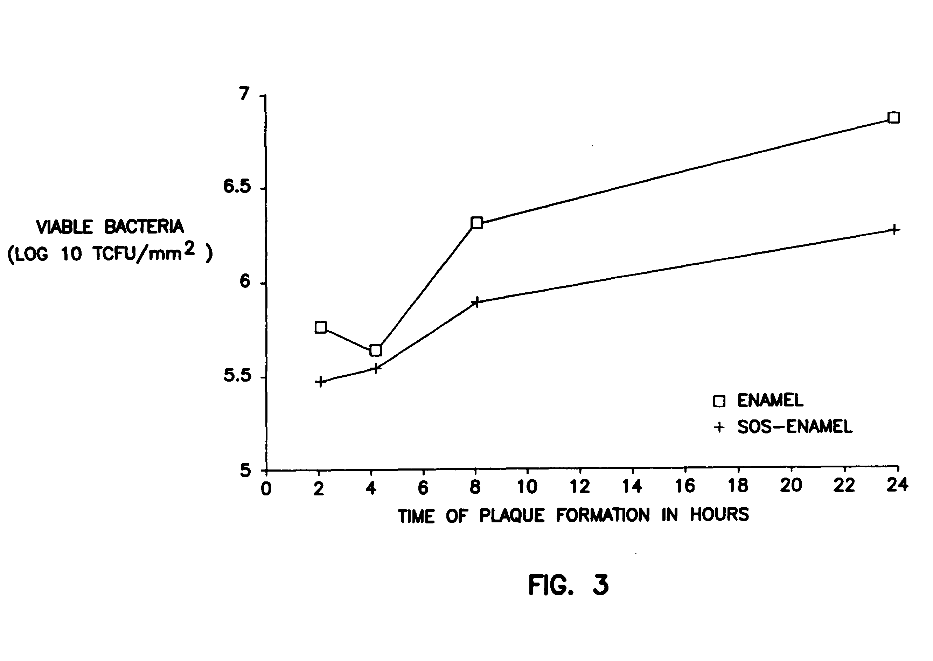

The effect of SOS treated enamel on in vivo plaque formation was compared to enamel in two subjects using a random placement of 3 pieces of enamel and 3 pieces of SOS-enamel in custom enclosures as described in Example 1. Each subject was examined twice for plaque formation after 2, 4, 8, andn 24 hours. Total viable bacteria were enumerated on the enamel and SOS-enamel as described in Example 1. Greatly lower viable bacteria (geometric mean) were found on SOS-enamel versus enamel after 2, 4, 8, and 24 hours, as illustrated in Fig. 3.

**TABLE 2**

| ENAMEL | | | |
|---|---|---|---|
| Angle | CYCLE | | |
| | 1 | 2 | 3 |
| Ad | 40 | 0 | 0 |
| Re | 0 | 0 | 0 |
| | | | |
| Ad | 64 | 35 | 29 |
| Re | 0 | 0 | 0 |

| SOS-ENAMEL | | | |
|---|---|---|---|
| Angle | CYCLE | | |
| | 1 | 2 | 3 |
| Ad | 103 | 71 | 56 |
| Re | 13 | 19 | 22 |
| | | | |
| Ad | 103 | 60 | 60 |
| Re | 28 | 33 | 34 |

| SC-SOS-ENAMEL | | | |
|---|---|---|---|
| Angle | CYCLE | | |
| | 1 | 2 | 3 |
| Ad | 57 | 0 | 0 |
| Re | 0 | 0 | 0 |
| | | | |
| Ad | 68 | 51 | 49 |
| Re | 33 | 34 | 36 |

### EXAMPLE 4 - Control of Population Density Using Hydrophobic Materials

Bacterial colonization of teeth in the mouth of 8 adults was also studied using a similar in vivo model. The 8 adult subjects used in this study were dental students or professional microbiologists. Pieces of bovine enamel, SILUX (trademark) restorative (3M, St. Paul, MN), VISIO-DISPERS (trademark) Iight-cured composite dental restorative (ESPE-Premier, Norristown, PA) (each piece having an area of about 10 mm²) were directly and concurrently fixed to 6 contralateral premolars and first molars, within custom enclosures, and left for varying time periods to accumulate dental plaque bacteria.

Bovine enamel pieces were cut into squares from extracted bovine teeth as described in Example 1 above. The composite resin pieces, VISIO-DISPERS (trademark) light-cured composite dental restorative (ESPE-Premier, Norristown, PA), and SILUX (trademark) restorative (3M, St. Paul, MN), were formed in a model made from EXPRESS vinyl polysiloxane registration material (3M, St. Paul, MN) in which a preformed bovine enamel piece was used to form a mold in the EXPRESS polysiloxane material. The composite material was applied to the preformed mold and a mylar strip was pressed over the top to form a smooth and even surface. The composite was then light cured for 40 seconds. The composite resin pieces were removed from the mold, the edges finished and the surface area measured. The outer surface of the pieces were polished with medium and fine SOF-LEX (trademark) disks (3M, St. Paul, MN) to simulate composite material applied in a clinical setting and to remove the resin rich layer.

Custom made piece enclosures were fabricated as described in Example 1. Cultivation of the various bacterial species was accomplished on a variety of elective and selective media as described in Example 1. Enamel pieces were removed at prescribed intervals and placed in 1.0 ml of PRAS diluent as described in Example 1 and sonified. The incorporation of [methyl-³H]-thymidine and [C¹⁴]-adenine into trichloroacetic acid insoluble material of the timed bacterial plaques was done as described in Example 1.

Fig. 4 illustrates that fewer pieces of VISIO-DISPERS (trademark) (ESPE-Premier, Norristown, PA) or SILUX (trademark) (3M, St. Paul, MN) showed a rapid increase of actively growing bacteria because fewer pieces approached the population density associated with stimulated or active bacterial growth. These surfaces were more hydrophobic than enamel when examined using the Wilhelmy balance for relative hydrophobicity and saliva-coated VISIO-DISPERS (trademark) (ESPE, Norristown, PA.) and saliva-coated SILUX (trademark) (3M, St. Paul, MN) were also more hydrophobic than saliva-coated enamel. Specifically, the mean (±SE) receding angle measurements of 8 replicates were as follows: Enamel 16.1 (±2.3), SC-Enamel 4.99 (±2.6), VISIO-DISPERS (trademark) composite 23.5 (±0.64), SC-VISIO-DISPERS (trademark) composite 14.1 (±2.3), SILUX (trademark) restorative 29.2 (±2.7), SC-SILUX (trademark) restorative 14.6 (±0.89). Saliva coating of all surfaces were significantly less hydrophobic than the uncoated parent surface (paired t-test). Additionally SC-SILUX (trademark) restorative particles were significantly more hydrophobic than SC-Enamel, repeated measures ANOVA, Scheffe f test. It was unexpected to find this hydrophobic "shine through", and that the deposited salivary pellicle was presumably modified to mediate a significant part of its hydrophobicity.

## Claims

1. A method of predicting when bacteria adhered to a surface will exhibit active growth, comprising either
(i) determining a population density of viable bacteria on the surface, comparing this population density with a population density of a population having a stimulated or enhanced proportion of actively growing bacteria to viable bacteria, and predicting bacterial growth when the first population density is greater than the second population density; or
(ii) determining a population density of viable bacteria on the surface, determining a population of actively growing bacteria on the surface, computing a ratio of actively growing bacteria to viable bacteria, and predicting active bacterial growth when said ratio is in the range of from about (5 x 10⁻⁴) : 1 to (20 x 10⁻²) : 1.

2. The method of claim 1 sub (i), wherein said population density of a population having a stimulated or enhanced proportion of actively growing bacteria to viable bacteria is from about 400,000 bacteria/mm² to about 6,300,000 bacteria/mm².

3. The method of claim 2 wherein said population density is from about 2,500,000 bacteria/mm² to about 3,200,000 bacteria/mm².

4. The method of claim 1 wherein the growth of bacteria occurs on a surface susceptible to bacterial infestation.

5. The method of claim 1 wherein the growth of bacteria occurs on the surface of a dental implant.

6. The method of claim 1 wherein the growth of bacteria occurs on an implanted biomaterial.

7. The method of claim 1 sub (ii), wherein active bacterial growth is predicted when said ratio is in the range of from about (10 x 10⁻⁴) : 1 to (40 x 10⁻⁴) : 1.

8. The method of claim 1 sub (ii), wherein the population of actively growing bacteria is determined by adding an excess of a labelled nucleoside to the bacteria for a time less than the minimum cell division time of the bacteria, growing the bacteria for an amount of time sufficient to allow bacterial incorporation of the labelled nucleotide and identifying the amount of labelled nucleotide incorporated into the DNA of the bacteria.

9. The method of claim 8 wherein the labelled nucleoside is selected from the group consisting of radiolabelled purines and pyrimidines.

10. The method of claim 8 wherein the labelled nucleoside is selected from the group consisting of [methyl-³H]-thymidine and [¹⁴C]-adenine and mixtures thereof.

11. A method of inhibiting bacterial growth on a surface comprising controlling the population of bacteria on the surface by maintaining the population density of the bacteria below a population density associated with an enhancement or stimulation of the proportion of growing cells to viable cells in the population.

12. The method of claim 11 wherein the population density of the bacteria on the surface is controlled by limiting the contact of adherence promoting agents with the bacteria.

13. The method of claim 12 wherein the adherence promoting agent is selected from the group consisting of polypeptides, glycoproteins and carbohydrates.

14. The method of claim 11 wherein the population density of the bacteria on the surface is controlled by modifying the hydrophobicity of the surface with an adherence suppressing agent.

15. The method of claim 14 wherein the adherence suppressing agent is sodium octadecyl sulfate.

16. The method of claim 11 wherein the population density of the bacteria on the surface is controlled by selecting a material having a mean dynamic contact advancing angle greater than about 60 degrees and a mean dynamic contact receding angle greater than about 20 degrees, said dynamic contact angles being measurable by using a Wilhelmy balance.

17. The method of claim 16 wherein the mean dynamic contact advancing angle is greater than 90 degrees.

18. The method of claim 11 wherein bacterial growth occurs in the presence of an aqueous fluid selected from the group consisting of body fluids, sera, carbohydrate-containing fluids, polypeptide-containing fluids, glycoprotein-containing fluids, or water.

19. The method of claim 11 wherein said population density associated with active growth is in the range of from about 400,000 to 6,300,000 bacteria/mm².

20. The method of claim 19 wherein said population density associated with active growth is in the range of from about 2,500,000 to 3,200,000 bacteria/mm².

21. A method of promoting bacterial growth on a surface comprising controlling the population of bacteria on the surface by maintaining the population density of the bacteria above a population density associated with an enhancement or stimulation of the proportion of growing cells to viable cells in the population.

## Patentansprüche

1. Verfahren zur Vorhersage des Zeitpunkts des aktiven Wachstums von Bakterien, die an einer Oberfläche haften, wobei das Verfahren umfaßt: entweder
(i) Bestimmen einer Populationsdichte von lebensfähigen Bakterien auf der Oberfläche, Vergleichen dieser Populationsdichte mit einer Populationsdichte einer Population mit einem stimulierten oder erhöhten Anteil von aktiv wachsenden Bakterien zu lebensfähigen Bakterien und Vorhersagen des Bakterienwachstums, sobald die erste Populationsdichte größer als die zweite Populationsdichte ist; oder
(ii) Bestimmen einer Populationsdichte von lebensfähigen Bakterien auf der Oberfläche, Bestimmen einer Population von aktiv wachsenden Bakterien auf der Oberfläche, Berechnen des Verhältnisses von aktiv wachsenden Bakterien zu lebensfähigen Bakterien und Vorhersagen des aktiven Bakterienwachstums, sobald das Verhältnis im Bereich von etwa (5 x 10⁻⁴) : 1 bis (20 x 10⁻²) : 1 liegt.

2. Verfahren nach Anspruch 1 unter (i), wobei die Populationsdichte einer Population mit einem stimulierten oder erhöhten Anteil von aktiv wachsenden Bakterien zu lebensfähigen Bakterien von etwa 400 000 Bakterien/mm² bis etwa 6.300 000 Bakterien/mm² reicht

3. Verfahren nach Anspruch 2, wobei die Populationsdichte von etwa 2.500.000 Bakterien/mm² bis etwa 3.200.000 Bakterien/mm² reicht.

4. Verfahren nach Anspruch 1, wobei das Bakterienwachstum auf einer Oberfläche erfolgt, die für Bakterienbefall empfänglich ist.

5. Verfahren nach Anspruch 1, wobei das Bakterienwachstum auf der Oberfläche eines Zahnimplantats erfolgt.

6. Verfahren nach Anspruch 1, wobei das Bakterienwachstum auf implantiertem Biomaterial erfolgt.

7. Verfahren nach Anspruch 1 unter (ii), wobei das aktive Bakterienwachstum vorhergesagt wird, sobald das Verhältnis im Bereich von etwa (10 x 10⁻⁴) : 1 bis (40 x 10⁻⁴) : 1 liegt.

8. Verfahren nach Anspruch 1 unter (ii), wobei die Population von aktiv wachsenden Bakterien bestimmt wird durch Zugeben eines Überschusses an markiertem Nucleosid zu den Bakterien für eine Zeit, die kürzer ist als die minimale Zellteilungszeit der Bakterien, Wachsenlassen der Bakterien für eine Zeitdauer, die ausreichend ist, um eine Einverleibung des markierten Nucleotids in die Bakterien zu erlauben, und Identifizieren der Menge an der DNA der Bakterien einverleibtem, markiertem Nucleotid.

9. Verfahren nach Anspruch 8, wobei das markierte Nucleosid aus der Gruppe bestehend aus radioaktiv markierten Purinen und Pyrimidinen ausgewählt wird.

10. Verfahren nach Anspruch 8, wobei das markierte Nucleosid aus der Gruppe bestehend aus [Methyl-³H]-thymidin und [¹⁴C]-Adenin und Gemischen davon ausgewählt wird.

11. Verfahren zur Inhibierung von Bakterienwachstum auf einer Oberfläche, umfassend das Steuern bzw. Kontrollieren der Bakterienpopulation auf der Oberfläche durch Aufrechterhalten der Populationsdichte der Bakterien unterhalb einer Populationsdichte, die mit einer Erhöhung oder Stimulation des Anteils von wachsenden Zellen zu lebensfähigen Zellen in der Population verbunden ist.

12. Verfahren nach Anspruch 11, wobei die Populationsdichte der Bakterien auf der Oberfläche gesteuert bzw. kontrolliert wird, indem man den Kontakt von das Anhaften fördernden Mitteln mit den Bakterien beschränkt.

13. Verfahren nach Anspruch 12, wobei das das Anhaften fördernde Mittel aus der Gruppe bestehend aus Polypeptiden, Glycoproteinen und Kohlenhydraten ausgewählt wird.

14. Verfahren nach Anspruch 11, wobei die Populationsdichte der Bakterien auf der Oberfläche gesteuert bzw. kontrolliert wird, indem man die Hydrophobie der Oberfläche mit einem das Anhaften unterdrückenden Mittel modifiziert.

15. Verfahren nach Anspruch 14, wobei das das Anhaften unterdrückende Mittel Natriumoctadecylsulfat ist.

16. Verfahren nach Anspruch 11, wobei die Populationsdichte der Bakterien auf der Oberfläche gesteuert bzw. kontrolliert wird, indem man ein Material mit einem mittleren dynamischen Kontaktwinkel beim Ausbreiten größer als etwa 60 Grad und einem mittleren dynamischen Kontaktwinkel beim Zurückweichen größer als etwa 20 Grad auswählt, wobei die dynamischen Kontaktwinkel unter Verwendung einer Wilhelmy-Waage meßbar sind.

17. Verfahren nach Anspruch 16, wobei der mittlere dynamische Kontaktwinkel beim Ausbreiten größer als 90 Grad ist.

18. Verfahren nach Anspruch 11, wobei das Bakterienwachstum in Gegenwart eines wäßrigen Fluids, ausgewählt aus der Gruppe bestehend aus Körperflüssigkeiten, Seren, Kohlenhydrat-haltigen Fluiden, Polypeptid-haltigen Fluiden, Glycoprotein-haltigen Fluiden oder Wasser erfolgt.

19. Verfahren nach Anspruch 11, wobei die mit aktivem Wachstum verbundene Populationsdichte im Bereich von etwa 400.000 bis 6.300.000 Bakterien/mm² liegt.

20. Verfahren nach Anspruch 19, wobei die mit aktivem Wachstum verbundene Populationsdichte im Bereich von etwa 2500.000 bis 3.200.000 Bakterien/mm² liegt.

21. Verfahren zur Förderung von Bakterienwachstum auf einer Oberfläche, umfassend das Steuern bzw. Kontrollieren der Bakterienpopulation auf der Oberfläche durch Aufrechterhalten der Populationsdichte der Bakterien oberhalb einer Populationsdichte, die mit einer Erhöhung oder Stimulation des Anteils von wachsenden Zellen zu lebensfähigen Zellen in der Population verbunden ist.

## Revendications

1. Procédé pour prédire quand des bactéries collées à une surface vont présenter une croissance active, ledit procédé comprenant soit
(i) la détermination d'une densité de population de bactéries viables sur la surface, la comparaison de cette densité de population avec celle d'une population ayant une proportion stimulée ou accrue de bactéries en croissance active par rapport aux bactéries viables, et la prévision de la croissance bactérienne lorsque la première densité de population est supérieure à la seconde densité de population ; soit
(ii) la détermination d'une densité de population de bactéries viables sur la surface, la détermination d'une population de bactéries en croissance active sur la surface, le calcul d'un rapport des bactéries en croissance active aux bactéries viables, et la prévision de la croissance active des bactéries lorsque ledit rapport se situe dans la gamme d'environ (5 x 10⁻⁴):1 à (20 x 10⁻²):1.

2. Procédé selon la revendication 1 sous (i), dans lequel ladite densité de population d'unc population ayant une proportion stimulée ou accrue de bactéries en croissance active par rapport aux bactéries viables est d'environ 400 000 bactéries/mm² à environ 6 300 000 bactéries/mm².

3. Procédé selon la revendication 2, dans lequel ladite densité de population est d'environ 2 500 000 bactéries/mm² à environ 3 200 000 bactéries/mm².

4. Procédé selon la revendication 1, dans lequel la croissance des bactéries a lieu sur une surface sensible à l'infestation bactérienne.

5. Procédé selon la revendication 1, dans lequel la croissance des bactéries a licu sur la surface d'un implant dentaire.

6. Procédé selon la revendication 1, dans lequel la croissance des bactéries a lieu sur un biomatériau implanté.

7. Procédé selon la revendication 1 sous (ii), dans lequel la croissance bactérienne active est prévisible lorsque ledit rapport se situe dans la gamme d'environ (10 x 10⁻⁴):1 à (40 x 10⁻⁴):1.

8. Procédé selon la revendication 1 sous (ii), dans lequel la population de bactéries en croissance active est déterminée en ajoutant aux bactéries un excès d'un nucléoside marqué pendant une durée inférieure à la durée minimale de division cellulaire des bactéries, en faisant croître les bactéries pendant une durée suffisante pour permettre l'incorporation du nucléotide marqué par les bactéries et, en déterminant la quantité de nucléotides marqués incorporés dans l'ADN des bactéries.

9. Procédé selon la revendication 8, dans lequel le nucléoside marqué est choisi dans le groupe constitué par les purines et les pyrimidines radiomarquées.

10. Procédé selon la revendication 8, dans lequel le nucléoside marqué est choisi dans le groupe constitué par la [méthyl-³H]-thymidine et la [¹⁴C]-adénine ainsi que leurs mélanges.

11. Procédé d'inhibition de la croissance bactérienne sur une surface, comprenant l'étape qui consiste à maîtriser la population de bactéries sur la surface en maintenant la densité de population des bactéries en dessous d'une densité de population associée à un accroissement ou à une stimulation de la proportion des cellules en croissance par rapport aux cellules viables dans la population.

12. Procédé selon la revendication 11, dans lequel la densité de population des bactéries sur la surface est maîtrisée en limitant le contact des agents d'activation de l'adhérence avec les bactéries.

13. Procédé selon la revendication 12, dans lequel l'agent d'activation de l'adhérence est choisi dans le groupe constitué par les polypeptides, les glycoprotéines et les glucides.

14. Procédé selon la revendication 11, dans lequel la densité de population des bactéries sur la surface est maîtrisée en modifiant l'hydrophobie de la surface à l'aide d'un agent de suppression de l'adhérence.

15. Procédé selon la revendication 14, dans lequel l'agent de suppression de l'adhérence est l'octadécylsulfate de sodium.

16. Procédé selon la revendication 11, dans lequel la densité de population des bactéries sur la surface est maîtrisée grâce au choix d'un matériel ayant un angle ascendant de contact dynamique moyen supérieur à environ 60 degrés et un angle descendant de contact dynamique moyen supérieur à environ 20 degrés, lesdits angles de contact dynamique étant mesurables à l'aide d'une balance de Wilhelmy.

17. Procédé selon la revcndication 16, dans lequel l'angle ascendant de contact dynamique moyen est supérieur à 90 degrés.

18. Procédé selon la revendication 11, dans lequel la croissance bactérienne a lieu en présence d'un fluide aqueux choisi dans le groupe constitué par les fluides corporels, les sérums, les fluides contenant des glucides, les fluides contenant des polypeptides, les fluides contenant des glycoprotéines, ou l'eau.

19. Procédé selon la revendication 11, dans lequel ladite densité de population associée à la croissance active est comprise dans la gamme d'environ 400 000 à 6 300 000 bactéries/mm².

20. Procédé selon la revendication 19, dans lequel ladite densité de population associée à la croissance active est comprise dans la gamme d'environ 2 500 000 à 3 200 000 bactéries/mm².

21. Procédé pour favoriser la croissance bactérienne sur une surface, comprenant l'étape qui consiste à maîtriser la population de bactéries sur la surface en maintenant la densité de population des bactéries au-dessus d'une densité de population associée à un accroissement ou à une stimulation de la proportion de cellules en croissance par rapport aux cellules viables dans la population.
